(19) Europäisches Patentamt
European Patent Office
Office européen des brevets

(11) **EP 4 088 755 B1**

(12) **EUROPEAN PATENT SPECIFICATION**

(45) Date of publication and mention
of the grant of the patent:
**30.07.2025 Bulletin 2025/31**

(21) Application number: **20963742.0**

(22) Date of filing: **08.12.2020**

(51) International Patent Classification (IPC):
*A61L 15/22* (2006.01)    *A61L 15/32* (2006.01)
*A61L 15/28* (2006.01)    *A61F 13/00* (2024.01)

(52) Cooperative Patent Classification (CPC):
(C-Sets available)
**A61F 13/01012; A61L 15/225**          (Cont.)

(86) International application number:
**PCT/KR2020/017838**

(87) International publication number:
**WO 2022/114348 (02.06.2022 Gazette 2022/22)**

(54) **COLLAGEN-ARGINATE WOUND DRESSING MATERIAL AND METHOD FOR PRODUCING SAME**

KOLLAGEN-ARGINAT-WUNDVERBANDMATERIAL UND VERFAHREN ZUM PRODUZIEREN
DESSELBEN

MATIÈRE DE PANSEMENT À BASE DE COLLAGÈNE-ARGINATE ET SON PROCÉDÉ DE
PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2020  KR 20200164194**

(43) Date of publication of application:
**16.11.2022  Bulletin 2022/46**

(73) Proprietor: **Plmicromed Co., Ltd.
Yangsan-si, Gyeongsangnam-do 50620 (KR)**

(72) Inventor: **LIM, Ju-sop
Yangsan-si, Gyeongsangnam-do 50595 (KR)**

(74) Representative: **Manitz Finsterwald
Patent- und Rechtsanwaltspartnerschaft mbB
Martin-Greif-Straße 1
80336 München (DE)**

(56) References cited:
WO-A1-2017/122216    CN-A- 107 661 229
CN-A- 108 014 366    CN-A- 109 847 097
KR-A- 20160 114 448    KR-A- 20180 050 954
KR-A- 20180 050 954    KR-B1- 101 144 493
KR-B1- 101 649 792    US-A1- 2017 182 209
US-A1- 2017 204 136

(52) Cooperative Patent Classification (CPC): (Cont.)

C-Sets
**A61L 15/225, C08L 5/04;**
**A61L 15/225, C08L 89/06**

**Description**

**TECHNICAL FIELD**

The present disclosure relates to a collagen-alginate wound dressing, including a matrix in which a low-molecularized collagen and a low-molecularized alginate are crosslinked and a method of preparing the same.

**BACKGROUND**

A wound dressing is a medical material designed for the purpose of suppressing contamination, protecting the skin, absorbing exudate, and suppressing bleeding or loss of body fluid of the wound site. The wound dressing can absorb a large amount of exudate from the wound site in a short time (absorptivity) and provide a moist environment onto the wound surface (wettability), and thus can effectively induce wound healing. In particular, a collagen wound dressing requires the use of living body-derived absorbent collagen and thus has relatively high bioresorbability and biocompatibility. Also, an alginate wound dressing is excellent in absorption of exudate, can maintain a moist environment in the wound site by forming a gel, and can minimize bacterial infection due to its unique antibacterial effect.

However, the conventional collagen wound dressing is made of high molecular weight collagen and thus has a low absorptivity. Therefore, in the case of a bed sore or a severely damaged wound, the conventional collagen wound dressing may less absorb exudate and may exhibit less antibacterial and anti-inflammatory activity. Also, the conventional collagen wound dressing is made of collagen derived from pigs or cows. Further, since the conventional alginate wound dressing does not have its own adhesive force, a separate secondary dressing is needed to fix it on the wound site. Furthermore, the conventional alginate wound dressing is not suitable for dry wounds or wounds with necrotic tissue. Moreover, the conventional alginate wound dressing causes the formation of fibrinous adhesion on the wound site, which may result in severe pain when it is removed. Besides, alginate in gel state is likely to be confused with pus or necrosis. When alginate is low-molecularized, it can be improved in antibacterial activity. However, the conventional alginate wound dressing is made of a polymer with 250 kDa in average molecular weight and thus needs to be low-molecularized.

In this regard, there have been attempts to low-molecularize collagen and alginate. However, the conventional chemical hydrolysis is performed by adding an acidic or basic material as a catalyst, and thus, there is a risk of environmental pollution and violent reaction and it is not suitable for commercialization as a medical material. Also, the enzymatic hydrolysis requires a relatively long reaction time and has a low efficiency, and may be accompanied with contamination during an enzyme removal process, which makes mass production difficult.

Korean Patent Laid-open Publication No. 10-2016-0087033 (Prior Art 1) discloses a collagen wound dressing that can carry a drug, but it contains a crosslinked product of simply physically pulverized collagen and uses a drug for wound healing, which is clearly different from the present disclosure.

Korean Patent Laid-open Publication No. 10-2017-0045458 (Prior Art 2) discloses a cell carrier for skin tissue regeneration in which fish-derived collagen, alginate and chitooligosaccharide are crosslinked. The cell carrier is a physical scaffold designed to enable culture in vitro and implantation of tissue cells, which is different in use from the present disclosure. Also, according to Prior Art 2, collagen and alginate are not low-molecularized, and high molecular weight chitooligosaccharide has rough particles and may cause damage to the wound site, and low molecular weight chitooligosaccharide loses antibacterial properties. Therefore, Prior Art 2 is clearly different from the present disclosure. US 2017/0204136 A1 discloses a polyelectrolyte based bioactive superabsorbent material ionotropically crosslinked/-neutralized involving polyvalent carboxylic acids, citrate, Kojic Acid, Alpha Arbutin along with fish scale collagen cross linked with other polyelectrolyte biopolymers preferably selected from chitosan, alginate or their combinations. US 2017/0182209 A1 discloses interpenetrating network hydrogels with independently tunable stiffness enhance tissue regeneration and wound healing. CN 108014366 A discloses a marine biological material composite hydrogel dressing, characterized in that it comprises a sodium alginate - fish collagen mixed solution and a chitosan - cross-linking agent mixed solution. CN 107661229 A discloses a chitosan and fish skin collagen peptide skin dressing, characterized by a mass composition as follows: Tilapia collagen peptide and sodium alginate 30 - 50%, carboxymethyl chitosan 40 - 60%, calcium chloride 1 - 5%, glycerin 1 - 5%, balance water, wherein the mass ratio of tilapia collagen peptide to sodium alginate mass ratio is 4:1-1:4. CN 109847097 A discloses a natural biological repairing membrane, characterized in that the raw material is water-soluble chitosan, alginate, fish collagen peptide and auxiliary materials.

**DISCLOSURE OF THE INVENTION**

**PROBLEMS TO BE SOLVED BY THE INVENTION**

The present disclosure provides a collagen-alginate wound dressing, including a matrix in which a low-molecularized collagen and a low-molecularized alginate are crosslinked and a method of preparing the same.

However, problems to be solved by the present disclosure are not limited to the above-described problems. Although not described herein, other problems to be solved by the present disclosure can be clearly understood by those skilled in the art from the following descriptions.

## MEANS FOR SOLVING THE PROBLEMS

A first aspect of the present disclosure provides a collagen-alginate wound dressing, including a matrix in which a low-molecularized collagen and a low-molecularized alginate are crosslinked, wherein an average molecular weight of the low-molecularized collagen is 10,000 Da to 30,000 Da, and an average molecular weight of the low-molecularized alginate is 5,000 Da to 30,000 Da.

A second aspect of the present disclosure provides a method of preparing a collagen-alginate wound dressing, including (a) mixing a low-molecularized collagen and a low-molecularized alginate to prepare a reaction mixture; and (b) adding a crosslinking agent to the reaction mixture and crosslinking the low-molecularized collagen and the low-molecularized alginate to obtain the collagen-alginate wound dressing according to the first aspect, wherein an average molecular weight of the low-molecularized collagen is 10,000 Da to 30,000 Da, and an average molecular weight of the low-molecularized alginate is 5,000 Da to 30,000 Da.

## EFFECTS OF THE INVENTION

A collagen-alginate wound dressing according to embodiments of the present disclosure can be used more effectively for wounds due to the properties of collagen that has excellent biocompatibility and biodegradability and alginate that exhibits an anti-inflammatory activity and protects the wounds against pathogenic infection.

The collagen-alginate wound dressing according to embodiments of the present disclosure has excellent absorption capacity for exudate and adequate moisture content to maintain a moist environment. Therefore, it can decrease the time of wound recovery and have excellent wound healing effect.

Collagen contained in the collagen-alginate wound dressing according to embodiments of the present disclosure is fish-derived with an average molecular weight of about 126 kDa, and due to its lower molecular weight than collagen extracted from pigskin or cowhide with an average molecular weight of about 300 kDa, in vivo permeability can be improved. The wound dressing according to embodiments of the present disclosure contains low-molecularized collagen and thus can activate cell regeneration by supplying nutrients, such as amino acids, to the wound site. Therefore, it can increase the wound healing effect.

The collagen-alginate wound dressing according to embodiments of the present disclosure uses fish-derived collagen and thus eliminates risks of infections, such as mad cow disease and foot-and-mouth disease, and can be used in the Muslim Halal market.

Collagen and alginate contained in the collagen-alginate wound dressing according to embodiments of the present disclosure can further maximize in vivo permeability by reducing the average molecular weight through a subcritical water low-molecularization process. Also, a hydrolysis reaction of collagen and alginate in the subcritical water low-molecularization process does not use acids, alkalis or enzymes, which have been used as catalysts in the conventional process. Therefore, the collagen-alginate wound dressing has excellent safety as a medical material, and thus, potential risks can be excluded.

The collagen-alginate wound dressing according to embodiments of the present disclosure contains low molecular weight collagen crosslinked with low molecular weight alginate, which is difficult to maintain its shape when being crosslinked, and thus improves the shape maintenance properties.

The collagen-alginate wound dressing according to embodiments of the present disclosure includes a matrix in which low-molecularized collagen and low-molecularized alginate are crosslinked and thus can maintain the shape of the matrix, sponge or wound dressing for a longer time than a wound dressing in which collagen and alginate are simply physically mixed. Therefore, it is possible to extend the time for which the wound dressing function is maintained.

The collagen-alginate wound dressing according to embodiments of the present disclosure is a wound dressing prepared by crosslinking low-molecularized collagen, which maintains a wet state and is effective in healing the necrotic tissue and/or bed sore site, and low-molecularized alginate, which exhibits excellent antibacterial activity. In particular, the collagen-alginate wound dressing according to embodiments of the present disclosure exhibits excellent absorption capacity for exudate at the tissue necrosis site through a subcritical water low-molecularization process and has the advantages of low molecular weight collagen, which causes less adhesion to the tissue at the bed sore site, and low molecular weight alginate, which has ideal antibacterial activity, as a wound dressing while remarkably reducing the disadvantages of the conventional high molecular weight collagen wound dressings and high molecular weight alginate wound dressings.

## BRIEF DESCRIPTION OF THE DRAWINGS

**FIG. 1A** shows a result of GPC molecular weight measurement of a collagen raw material before subcritical water hydrolysis ($M_w$: 51,058; $M_n$: 2,534) according to an example of the present disclosure, and **FIG. 1B** shows a result of GPC average molecular weight measurement of low-molecularized collagen after subcritical water hydrolysis according to Example 1 of the present disclosure.

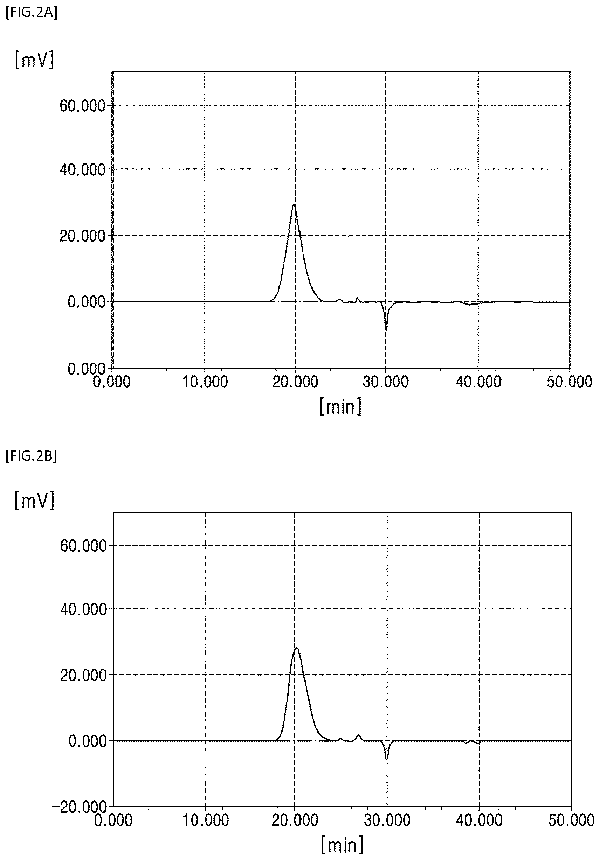

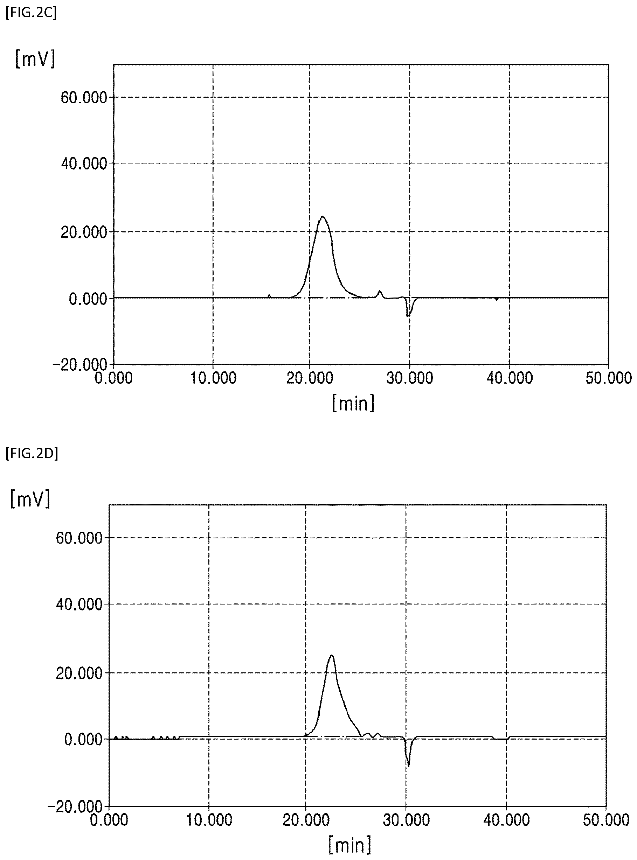

**FIG. 2A** to **FIG. 2D** show results of average molecular weight measurement of low-molecularized collagen after subcritical water hydrolysis depending on conditions of subcritical water hydrolysis according to an example of the present disclosure.

**FIG. 3** shows photographs of crosslinked collagen-alginate wound dressings prepared according to examples of the present disclosure.

**FIG. 4A** and **FIG. 4B** show FT-IR results before and after crosslinking between collagen and alginate.

**FIG. 5A** to **FIG. 5D** are SEM images before and after crosslinking between collagen and alginate.

## BEST MODE FOR CARRYING OUT THE INVENTION

Hereinafter, examples of the present disclosure will be described in detail with reference to the accompanying drawings so that the present disclosure may be readily implemented by those skilled in the art. However, it is to be noted that the present disclosure is not limited to the examples but can be embodied in various other ways. In drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element.

Through the whole document, the term "on" that is used to designate a position of one element with respect to another element includes both a case that the one element is adjacent to the other element and a case that any other element exists between these two elements.

Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

Through the whole document, the term "about or approximately" or "substantially" is intended to have meanings close to numerical values or ranges specified with an allowable error and intended to prevent accurate or absolute numerical values disclosed for understanding of the present disclosure from being illegally or unfairly used by any unconscionable third party.

Through the whole document, the term "step of" does not mean "step for".

Through the whole document, the term "combination of" included in Markush type description means mixture or combination of one or more components, steps, operations and/or elements selected from a group consisting of components, steps, operation and/or elements described in Markush type and thereby means that the disclosure includes one or more components, steps, operations and/or elements selected from the Markush group.

Through this whole specification, a phrase in the form "A and/or B" means "A or B, or A and B".

Throughout the whole document, the term "subcritical water" refers to high-temperature and high-pressure water above water's boiling point of about 100°C at about 0.1 MPa or more and below water's critical point of about 372.2°C at about 22.4 MPa or less.

Throughout the whole document, the term "average molecular weight" refers to "weight average molecular weight".

Hereinafter, the present disclosure will be described in more detail with reference to exemplary embodiments. However, the present disclosure is not limited to these exemplary embodiments.

A first aspect of the present disclosure provides a collagen-alginate wound dressing, including a matrix in which a low-molecularized collagen and a low-molecularized alginate are crosslinked.

The average molecular weight of the low-molecularized collagen is about 10,000 Da to about 30,000 Da. For example, the average molecular weight of the low-molecularized collagen may be about 10,000 Da to about 25,000 Da, about 10,000 Da to about 20,000 Da, about 10,000 Da to about 15,000 Da, about 15,000 Da to about 30,000 Da, about 15,000 Da to about 25,000 Da, about 15,000 Da to about 20,000 Da, about 20,000 Da to about 30,000 Da, about 20,000 Da to about 25,000 Da, or about 25,000 Da to about 30,000 Da. Herein, when the average molecular weight of the low-molecularized collagen is more than about 30,000 Da, the absorption capacity for exudate may decrease and the self-degradation time may increase. When the average molecular weight of the low-molecularized collagen is less than about 10,000 Da, crosslinking of the low-molecularized collagen and the low-molecularized alginate does not occur well, which is not suitable for preparation of a wound dressing.

The average molecular weight of the low-molecularized alginate is about 5,000 Da to about 30,000 Da. For example, the average molecular weight of the low-molecularized alginate may be about 5,000 Da to about 25,000 Da, about 5,000 Da to about 20,000 Da, about 5,000 Da to about 15,000 Da, about 5,000 Da to about 10,000 Da, about 10,000 Da to about 30,000 Da, about 10,000 Da to about 25,000 Da, about 10,000 Da to about 20,000 Da, about 10,000 Da to about 15,000 Da, about 15,000 Da to about 30,000 Da, about 15,000 Da to about 25,000 Da, about 15,000 Da to about 20,000 Da, about 20,000 Da to about 30,000 Da, about 20,000 Da to about 25,000 Da, or about 25,000 Da to about 30,000 Da. Herein, when the average molecular weight of the low-molecularized alginate is less than about 5,000 Da, crosslinking of alginate and collagen may not occur well, or may not be finally formed into a wound dressing. When the average molecular weight of the low-molecularized alginate is more than about 30,000 Da, the antibacterial and antioxidant effects of alginate may decrease.

In an exemplary embodiment of the present disclosure, the low-molecularized collagen and the low-molecularized alginate may be obtained by low-molecularizing a collagen and an alginate using subcritical water low-molecularization process, respectively. Herein, the collagen may be fish-derived collagen.

In an exemplary embodiment of the present disclosure, a weight ratio of the low-molecularized collagen and the low-molecularized alginate may be about 6:4 to about 9:1, but may not be limited thereto. In an exemplary embodiment of the present disclosure, the weight ratio of the low-molecularized collagen and the low-molecularized alginate may be about 7:3. Herein, when the weight ratio of the low-molecularized collagen and the low-molecularized alginate is less than about 6:4, the ratio of the low-molecularized collagen decreases, and, thus, in the preparation of the collagen-alginate wound dressing, crosslinking does not occur well, or the absorption capacity among the performances of the finally prepared wound dressing may decrease, which makes it difficult to provide a moist environment onto the wound or bed sore site. When the weight ratio of the low-molecularized collagen and the low-molecularized alginate is more than about 9:1, the ratio of the low-molecularized alginate decreases, and, thus, the antibacterial and antioxidant effects of the low-molecularized alginate may not be exhibited. However, the present disclosure may not be limited thereto.

In an exemplary embodiment of the present disclosure, an absorption capacity of the collagen-alginate wound dressing for exudate may be about 10 g/100 cm$^2$ or more, but may not be limited thereto. For example, the absorption capacity of the collagen-alginate wound dressing for exudate may be about 10 g/100 cm$^2$ or more, about 20 g/100 cm$^2$ or more, about 10 g/100 cm$^2$ to about 50 g/100 cm$^2$, about 10 g/100 cm$^2$ to about 40 g/100 cm$^2$, about 10 g/100cm$^2$ to about 30 g/100cm$^2$, about 20 g/100cm$^2$ to about 50 g/100cm$^2$, about 20 g/100cm$^2$ to about 40 g/100cm$^2$, or about 20 g/100cm$^2$ to about 30 g/100cm$^2$, but may not be limited thereto. In an exemplary embodiment of the present disclosure, the absorption capacity of the collagen-alginate wound dressing for exudate may be about 20 g/100 cm$^2$ to about 30 g/100 cm$^2$.

In an exemplary embodiment of the present disclosure, a moisture content of the collagen-alginate wound dressing may be about 18% or less, but may not be limited thereto. For example, the moisture content of the collagen-alginate wound dressing may be about 18% or less, about 16% or less, about 15% or less, about 14% or less, about 12% or less, about 10% or less, about 8% or less, about 6% or less, about 5% or less, about 4% or less, or about 2% or less, but may not be limited thereto.

A second aspect of the present disclosure provides a method of preparing a collagen-alginate wound dressing, including (a) mixing a low-molecularized collagen and a low-molecularized alginate to prepare a reaction mixture; and (b) adding a crosslinking agent to the reaction mixture and crosslinking the low-molecularized collagen and the low-molecularized alginate to obtain the collagen-alginate wound dressing according to the first aspect.

Detailed descriptions on the second aspect of the present disclosure, which overlap with those on the first aspect of the present disclosure, are omitted hereinafter, but the descriptions of the first aspect of the present disclosure may be identically applied to the second aspect of the present disclosure, even though they are omitted hereinafter.

In an exemplary embodiment of the present disclosure, the method, in (a), may further include obtaining the low-molecularized collagen and the low-molecularized alginate by subcritical water hydrolysis of a collagen and an alginate, respectively.

In an exemplary embodiment of the present disclosure, the subcritical water hydrolysis may be carried out under pressure of inert gas (He, Ar etc.), $N_2$ or $CO_2$, but may not be limited thereto. In an exemplary embodiment of the present disclosure, the subcritical water hydrolysis may be carried out under pressure of $N_2$ or $CO_2$.

In an exemplary embodiment of the present disclosure, the subcritical water hydrolysis may be carried out at a temperature range of about 110°C to about 150°C, but may not be limited thereto. For example, the subcritical water hydrolysis may be carried out at the temperature range of about 110°C to about 150°C, about 110°C to about 140°C, about 110°C to about 130°C, about 120°C to about 150°C, about 120°C to about 140°C, about 120°C to about 130°C, or about 130°C to about 150°C, but may not be limited thereto. In an exemplary embodiment of the present disclosure, the subcritical water hydrolysis may be carried out at the temperature range of about 110°C to about 150°C. In an exemplary embodiment of the present disclosure, the subcritical water hydrolysis may be carried out at the temperature range of about 110°C to about 130°C, or about 130°C to about 150°C.

In an exemplary embodiment of the present disclosure, the method, in (b), may further include crosslinking the low-molecularized collagen and the low-molecularized alginate and freeze-drying, but may not be limited thereto.

In an exemplary embodiment of the present disclosure, the crosslinking agent may be at least one selected from formaldehyde, glutaraldehyde, dialdehyde starch, 1-ethyl-3-(3-dimethylaminopropyl) carbodiimide (EDC), NHS, epoxy compound, glutaraldehyde glyoxal, glyoxal, 2,2-Dimethoxy-2-phenylacetophenone, sodium tripolyphosphate, diacetaldehyde PEG, scleraldehyde, diethyl squarate, epichlorohydrin genipin, tannins, phenylpropanoids, catechins, resveratrol, flavonoids, and isoflavonoids, but may not be limited thereto. In an exemplary embodiment of the present disclosure, the crosslinking agent may be formaldehyde, glutaraldehyde, dialdehyde starch, or 1-ethyl-3-(3-dimethylaminopropyl) carbodiimide (EDC).

Hereinafter, example embodiments are described in more detail by using Examples, but the present disclosure may not limited to the Examples.

## MODE FOR CARRYING OUT THE INVENTION

[EXAMPLES]

1. Preparation of collagen-alginate wound dressing

(1) Low-molecularization process of collagen

Fish-derived collagen was purchased from MSC and used, and low-molecularized collagen having a molecular weight of from 10,000 Da to 30,000 Da suitable for collagen-alginate wound dressing was obtained through subcritical water hydrolysis conducted for 20 minutes to 1 hour by pressurizing an inert gas containing $N_2$ and $CO_2$ at 30 bar to 60 bar in 1% (w/v) to 15% (w/v) collagen aqueous solution (deionized water) at 100°C to 150°C. The average molecular weight was confirmed by the KOPTRI, an accredited certification institute.

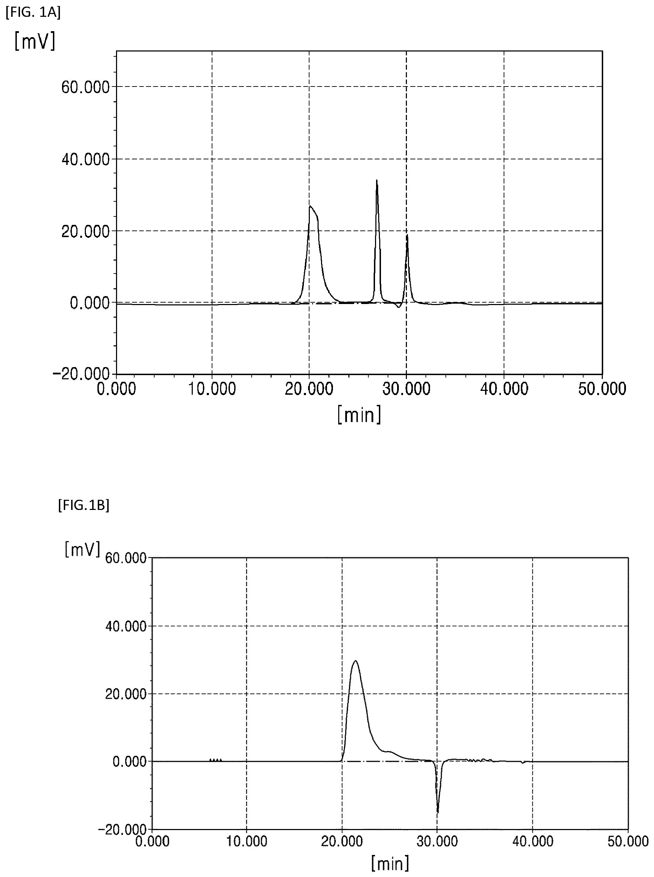

**FIG. 1A** shows a result of GPC molecular weight measurement of a collagen raw material before subcritical water hydrolysis ($M_w$: 51,058; $M_n$: 2,534), and **FIG. 1B** shows a result of GPC molecular weight measurement of low-molecularized collagen after subcritical water hydrolysis ($M_w$: 21,884; $M_n$: 6,822).

(2) Low-molecularization process of alginate

Sodium alginate purchased from Daejung Chemicals & Metals was used as an alginate raw material, and subcritical water hydrolysis was conducted at 30 bar to 60 bar for 20 minutes to 1 hour under the conditions described in Table 1 to obtain low-molecularized alginate.

[Table 1]

| Condition | Number-average molecular weight ($M_n$) | Weight-average molecular weight ($M_w$) | Confirmation of molecular weight |
|---|---|---|---|
| $N_2$, 110-130°C | 102,749 | 260,940 | FIG. 2A |
| $N_2$, 130-150°C | 76,777 | 189,478 | FIG. 2B |
| $CO_2$, 110-130°C | 28,412 | 88,290 | FIG. 2C |
| $CO_2$, 130-150°C | 10,173 | 23,389 | FIG. 2D |

(3) Preparation of collagen-alginate wound dressing

The low-molecularized collagen (average molecular weight: 21,885) and low-molecularized alginate (average molecular weight: 23,389) obtained through the above processes were used to prepare a collagen-alginate wound dressing. A 3% (w/v) low-molecularized aqueous collagen solution (deionized water) dispersed in deionized water and a 3% (w/v) low-molecularized aqueous alginate solution (deionized water) dispersed in deionized water were mixed at a mass ratio of solutes ranging from 6:4 to 7:3 with primary stirring at 60°C at 500 rpm for 1 hour to prepare a mixed solution. Thereafter, a crosslinking agent (formaldehyde, glutaraldehyde, EDC, NHS, etc.) with a concentration of 5 μL/mL was added to the mixed solution, followed by secondary stirring at 30°C at 100 rpm for 16 hours to prepare a crosslinked collagen-alginate aqueous solution in sol state. The obtained collagen-alginate aqueous solution was dispensed to a thickness of 5 mm into a mold and frozen at -20°C for 6 hours. Then, through lyophilization for 24 hours, a collagen-alginate wound dressing was prepared.

Sponges before crosslinking of the low-molecularized alginate used in the above process depending on conditions of subcritical water hydrolysis were denoted as A to D and wound dressings after crosslinking were denoted as A' to D', which are shown in Table 2 below:

[Table 2]

| Sponges before crosslinking | | Wound dressings after crosslinking | |
|---|---|---|---|
| A | Collagen/Alginate sponge | A' | Crosslinked Collagen/Alginate |
| B | Collagen/Subcritical water hydrolyzed alginate sponge at (100-120)°C | B' | Crosslinked Collagen/Subcritical water hydrolyzed alginate at (100-120)°C |
| C | Collagen/Subcritical water hydrolyzed alginate sponge at (110-130)°C | C' | Crosslinked Collagen/Subcritical water hydrolyzed alginate at (110-130)°C |
| D | Collagen/Subcritical water hydrolyzed alginate sponge at (130-150)°C | D' | Crosslinked Collagen/Subcritical water hydrolyzed alginate at (130-150)°C |

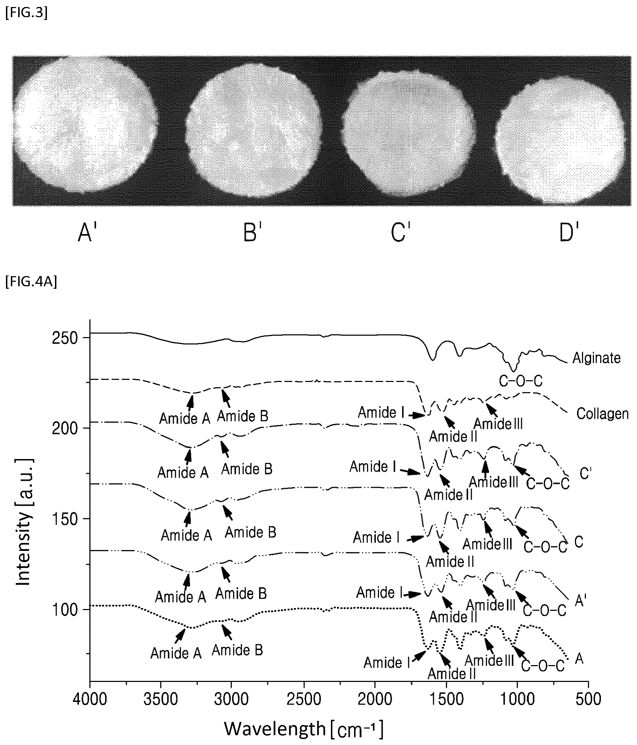

**FIG. 3** shows photographs of the crosslinked wound dressings A' to D' in order from left to right.

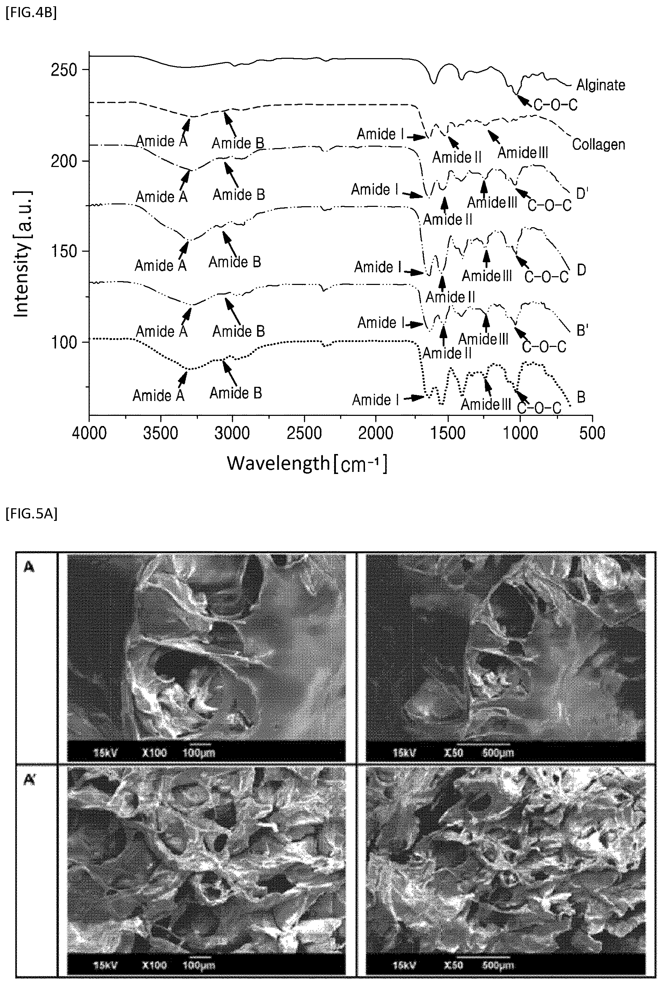

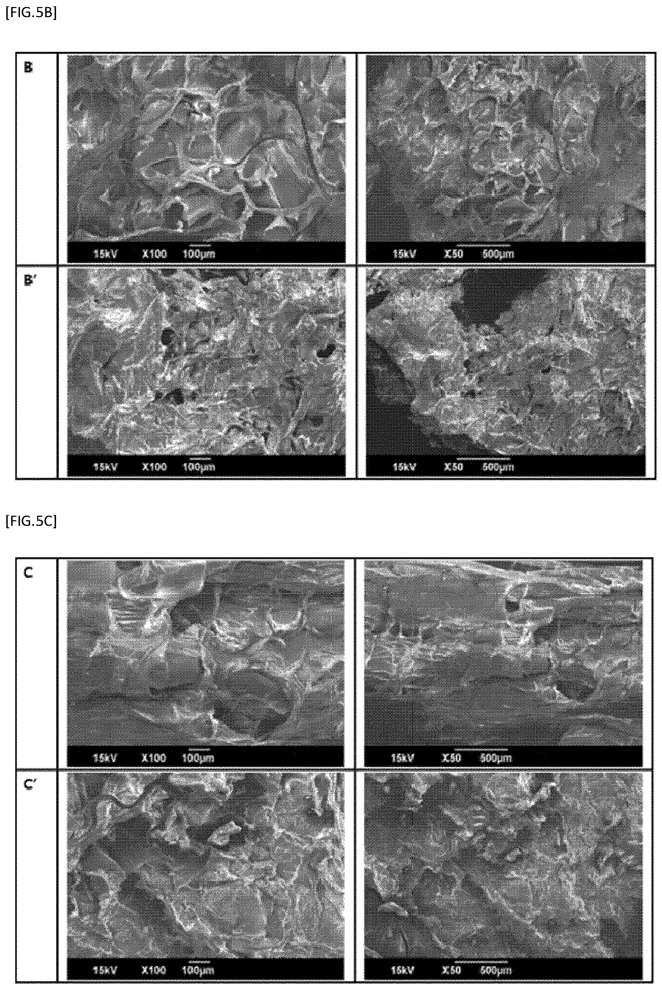

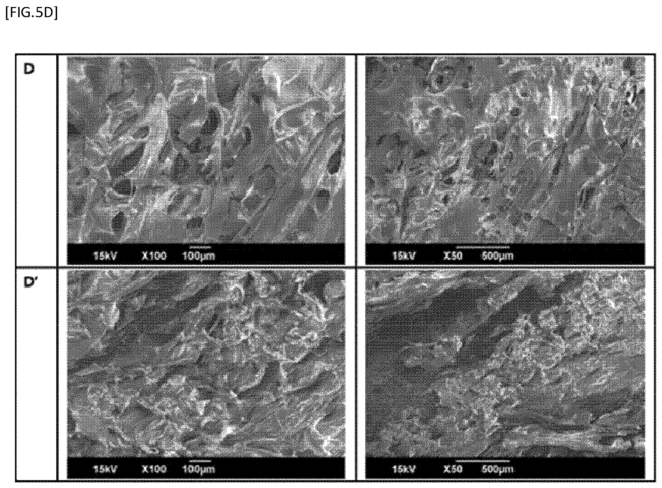

As can be seen from **FIG. 4A** and **FIG. 4B,** FT-IR results before and after crosslinking between collagen and alginate according to the above preparation process were obtained. As can be seen from **FIG. 5A** to **FIG. 5D,** SEM images before and after crosslinking between collagen and alginate according to the above preparation process were obtained. Referring to **FIG. 4** and **FIG. 5**, it was confirmed that crosslinking of collagen-alginate occurs by the above preparation process.

2. Efficacy test of collagen-alginate wound dressing

(1) Appearance test of wound dressing

An appearance test was conducted at the Korea Testing and Research Institute, a certification institute. The appearance of the wound dressing was visually checked for any external flaws, damage or foreign substances on the product. Even after the following absorption capacity and moisture content tests, the product was checked for external flaws, damage or foreign substances, etc.

(2) Absorption capacity test of wound dressing

The absorption capacity is the ability to absorb exudate from a bed sore or wound, and can be used as an index for maintaining a moist environment. The absorption capacity test was conducted at the Korea Testing and Research Institute, a certification institute.

The wound dressing (sample) of $(5 \times 5)$ cm$^2$ was placed in a Petri dish to measure a weight ($W_1$), and distilled water pre-warmed to $(37 \pm 1)$°C was measured accurately up to $\pm 0.5$ g and added in consideration of the absorption capacity of the product (for example, 40 times the weight of the sample). The sample was left in a $(37 \pm 1)$°C thermostat for 30 minutes and then hung with tweezers for 30 seconds to measure a weight ($W_2$). Then, an absorption capacity (g/cm$^2$) was measured according to Equation 1 below.

$$\text{Absorption capacity} = (W_1 - W_2) / \text{Area of the initial sample (Equation 1)}$$

As a result of the absorption capacity test, the prepared wound dressing showed an absorption capacity of 6.20 g/25 cm$^2$.

(3) Moisture content test of wound dressing

As the moisture content increases, the absorption capacity for exudate increases. Therefore, the moisture content can be used as an index for maintaining a moist environment. The moisture content test was conducted at the Korea Testing and Research Institute, a certification institute.

Before weighing, the weight of the empty measuring vessel dried at 102°C was measured. About 1.5 g of the quadrisected sample was placed in the measuring vessel, weighed, and dried at $(102 \pm 2)$°C for 5 hours. Thereafter, the vessel and contents were dried in a drying device for 30 minutes and weighed and repeatedly dried, cooled and weighed.

**EP 4 088 755 B1**

When the difference in weight did not exceed 3 mg, drying continues for 1 hour or drying was performed until the total drying time reached 8 hours. The final weight of the sample and the weight of the vessel were recorded, and the weight of the dried sample was calculated according to Equation 2 below.

$$\text{Moisture content} = [(m_1 - m_2) / m_2] \times 100 \quad \text{(Equation 2)}$$

$m_1$: Sample weight before drying (g)
$m_2$: Sample weight after drying (g)

As a result of the moisture content test, the prepared wound dressing showed a moisture content of 1.82%.

3. Antioxidant activity test of alginate

(1) Test on reducing power with respect to ferric cyanide ($[Fe(CN)_6]^{3-}$, $Fe^{3+}$)

The test was conducted according to the method of Ak T. and Gulcin. I. (2008). A set of alginate raw material without subcritical water hydrolysis and each of four sets of 0.5 mL of alginate hydrolyzate having a different average molecular weight depending on conditions of subcritical water hydrolysis were mixed with 1.25 mL of 0.2 M phosphate buffer (pH 6.6) and 1.25 mL of 1% (w/v) potassium ferricyanide aqueous solution and then reacted at 50°C for 30 minutes. Then, 1.25 mL of TCA (10%, w/v) was added thereto, followed by centrifugation at a speed of 3,000 rpm for 10 minutes. Thereafter, 1.25 mL of distilled water was added to 1.25 mL of the supernatant and 0.25 mL of 0.1% (w/v) ferric chloride aqueous solution was added, followed by reaction for 10 minutes. Then, the absorbance was measured at 700 nm.

As a result of the test, the reducing power of the alginate raw material with an average molecular weight of 250,000 without subcritical water hydrolysis was confirmed to be 46.50±0.41%. The average molecular weight was adjusted depending on conditions of subcritical water hydrolysis and the molecular weight of the alginate hydrolyzate used in the test decreased. Accordingly, the reducing power with respect to ferric cyanide increased from 64.06% to 88.86%, and the detailed results are shown in Table 3 below:

[Table 3]

|  |  | Pressurized gas | |
|---|---|---|---|
|  |  | $N_2$ | $CO_2$ |
| Hydrolysis temperature | 110-130°C | $M_n$: 102,749; $M_w$: 260,940 | $M_n$: 28,412; $M_w$: 88,290 |
|  |  | 64.06±0.19% | 74.44±0.59% |
|  | 130-150°C | $M_n$: 76,777; $M_w$: 189,478 | $M_n$: 10,173; $M_w$: 23,389 |
|  |  | 73.51±0.14% | 88.86±0.28% |

(2) Hydroxyl radical (OH) scavenging ability test

The test was conducted according to the method of Wei L. et al. (2010). A set of alginate raw material without subcritical water hydrolysis and each of four sets of 1 mL of alginate hydrolyzate having a different average molecular weight depending on conditions of subcritical water hydrolysis were mixed with 0.5 mL of 1.5 mM $FeSO_4$, 0.35 mL of 6 mM $H_2O_2$ and 0.15 mL of 20 mM sodium salicylate and then reacted at 37°C for 1 hour. Thereafter, the absorbance was measured at 562 nm.

As a result of the test, the hydroxyl radical scavenging ability of the alginate raw material with an average molecular weight of 250,000 without subcritical water hydrolysis was confirmed to be 81.51±0.71%. The average molecular weight was adjusted depending on conditions of subcritical water hydrolysis and the molecular weight of the alginate hydrolyzate used in the test decreased. Accordingly, the hydroxy radical scavenging ability increased from 90.52% to 93.75%, and the detailed results are shown in Table 4 below:

[Table 4]

| | | Pressurized gas | |
|---|---|---|---|
| | | $N_2$ | $CO_2$ |
| Hydrolysis temperature | 110-130°C | $M_n$: 102,749; $M_w$: 260,940 | $M_n$: 28,412; $M_w$: 88,290 |
| | | 90.52±0.51% | 92.47±0.46% |
| | 130-150°C | $M_n$: 76,777; $M_w$: 189,478 | $M_n$: 10,173; $M_w$: 23,389 |
| | | 91.36±0.08% | 93.75±0.08% |

The above description of the example embodiments is provided for the purpose of illustration, and it would be understood by those skilled in the art that various changes and modifications may be made without changing technical conception and essential features of the example embodiments. Thus, it is clear that the above-described example embodiments are illustrative in all aspects and do not limit the present disclosure. For example, each component described to be distributed can be implemented in a combined manner.

**Claims**

1.  A collagen-alginate wound dressing, comprising a matrix in which a low-molecularized collagen and a low-molecularized alginate are crosslinked,

    wherein an average molecular weight of the low-molecularized collagen is 10,000 Da to 30,000 Da, and
    wherein an average molecular weight of the low-molecularized alginate is 5,000 Da to 30,000 Da.

2.  The collagen-alginate wound dressing of Claim 1,
    wherein the low-molecularized collagen and the low-molecularized alginate are obtained by low-molecularizing a collagen and an alginate using subcritical water low-molecularization process, respectively.

3.  The collagen-alginate wound dressing of Claim 1,
    wherein a weight ratio of the low-molecularized collagen and the low-molecularized alginate is 6:4 to 9:1.

4.  The collagen-alginate wound dressing of Claim 1,
    wherein an absorption capacity of the collagen-alginate wound dressing for exudate is 10 g/100 cm$^2$ or more, wherein the absorption capacity is measured in accordance with the method of the description.

5.  The collagen-alginate wound dressing of Claim 1,
    wherein a moisture content of the collagen-alginate wound dressing is 18% or less, wherein the moisture content is measured in accordance with the method of the description.

6.  A method of preparing a collagen-alginate wound dressing, comprising:

    (a) mixing a low-molecularized collagen and a low-molecularized alginate to prepare a reaction mixture; and
    (b) adding a crosslinking agent to the reaction mixture and crosslinking the low-molecularized collagen and the low-molecularized alginate to obtain the collagen-alginate wound dressing of any one of Claims 1 to 5,

    wherein an average molecular weight of the low-molecularized collagen is 10,000 Da to 30,000 Da, and
    wherein an average molecular weight of the low-molecularized alginate is 5,000 Da to 30,000 Da.

7.  The method of Claim 6,
    in (a), further comprising obtaining the low-molecularized collagen and the low-molecularized alginate by subcritical water hydrolysis of a collagen and an alginate, respectively.

8.  The method of Claim 7,
    wherein the subcritical water hydrolysis is carried out under pressure of inert gas, $N_2$ or $CO_2$.

9.  The method of Claim 7,

wherein the subcritical water hydrolysis is carried out at a temperature range of 110°C to 150°C.

10. The method of Claim 6,
   in (b), further comprising crosslinking the low-molecularized collagen and the low-molecularized alginate and freeze-drying.

11. The method of Claim 6,
   wherein the crosslinking agent is at least one selected from formaldehyde, glutaraldehyde, dialdehyde starch, 1-ethyl-3-(3-dimethylaminopropyl) carbodiimide (EDC), NHS, epoxy compound, glutaraldehyde glyoxal, glyoxal, 2,2-Dimethoxy-2-phenylacetophenone, sodium tripolyphosphate, diacetaldehyde PEG, scleraldehyde, diethyl squarate, epichlorohydrin genipin, tannins, phenylpropanoids, catechins, resveratrol, flavonoids, and isoflavonoids.

**Patentansprüche**

1. Kollagen-Alginat-Wundverband, umfassend eine Matrix, in der ein niedermolekularisiertes Kollagen und ein niedermolekularisiertes Alginat vernetzt sind,

   wobei ein mittleres Molekulargewicht des niedermolekularisierten Kollagens 10.000 Da bis 30.000 Da beträgt, und
   wobei ein mittleres Molekulargewicht des niedermolekularisierten Alginats 5.000 Da bis 30.000 Da beträgt.

2. Kollagen-Alginat-Wundverband nach Anspruch 1,
   wobei das niedermolekularisierte Kollagen und das niedermolekularisierte Alginat erhalten sind durch Niedermolekularisieren eines Kollagens beziehungsweise eines Alginats unter Verwendung eines Niedermolekularisierungsprozesses mit subkritischem Wasser.

3. Kollagen-Alginat-Wundverband nach Anspruch 1,
   wobei ein Gewichtsverhältnis des niedermolekularisierten Kollagens und des niedermolekularisierten Alginats 6:4 bis 9:1 beträgt.

4. Kollagen-Alginat-Wundverband nach Anspruch 1,
   wobei eine Absorptionskapazität des Kollagen-Alginat-Wundverbands für Exsudat 10 g/100 cm$^2$ oder mehr beträgt, wobei die Absorptionskapazität gemäß dem Verfahren der Beschreibung gemessen ist.

5. Kollagen-Alginat-Wundverband nach Anspruch 1,
   wobei ein Feuchtigkeitsgehalt des Kollagen-Alginat-Wundverbands 18% oder weniger beträgt, wobei der Feuchtigkeitsgehalt gemäß dem Verfahren der Beschreibung gemessen ist.

6. Verfahren zur Herstellung eines Kollagen-Alginat-Wundverbands, umfassend:

   (a) Vermischen eines niedermolekularisierten Kollagens und eines niedermolekularisierten Alginats, um eine Reaktionsmischung herzustellen; und
   (b) Zugeben eines Vernetzungsmittels zu der Reaktionsmischung und Vernetzen des niedermolekularisierten Kollagens und des niedermolekularisierten Alginats, um den Kollagen-Alginat-Wundverband nach einem der Ansprüche 1 bis 5 zu erhalten,

   wobei ein mittleres Molekulargewicht des niedermolekularisierten Kollagens 10.000 Da bis 30.000 Da beträgt, und
   wobei ein mittleres Molekulargewicht des niedermolekularisierten Alginats 5.000 Da bis 30.000 Da beträgt.

7. Verfahren nach Anspruch 6,
   welches, in (a), zudem ein Erhalten des niedermolekularisierten Kollagens und des niedermolekularisierten Alginats durch Hydrolyse eines Kollagens beziehungsweise eines Alginats mit subkritischem Wasser umfasst.

8. Verfahren nach Anspruch 7,
   wobei die Hydrolyse mit subkritischem Wasser unter Druck von Inertgas, $N_2$ oder $CO_2$ durchgeführt wird.

**9.** Verfahren nach Anspruch 7,
wobei die Hydrolyse mit subkritischem Wasser bei einem Temperaturbereich von 110°C bis 150°C durchgeführt wird.

**10.** Verfahren nach Anspruch 6,
welches, in (b), zudem Vernetzen des niedermolekularisierten Kollagens und des niedermolekularisierten Alginats und Gefriertrocknen umfasst.

**11.** Verfahren nach Anspruch 6,
wobei das Vernetzungsmittel mindestens eines ist, welches ausgewählt ist aus Formaldehyd, Glutaraldehyd, Dialdehydstärke, 1-Ethyl-3-(3-dimethylaminopropyl)carbodiimid (EDC), NHS, Epoxyverbindung, Glutaraldehyd-Glyoxal, Glyoxal, 2,2-Dimethoxy-2-phenylacetophenon, Natriumtripolyphosphat, Diacetaldehyd-PEG, Scleraldehyd, Diethylquadratat, Epichlorhydrin-Genipin, Tanninen, Phenylpropanoiden, Catechinen, Resveratrol, Flavonoiden und Isoflavonoiden.

**Revendications**

**1.** Pansement à base de collagène-alginate, comprenant une matrice dans laquelle un collagène à poids moléculaire réduit et un alginate à poids moléculaire réduit sont réticulés,

dans lequel un poids moléculaire moyen du collagène à poids moléculaire réduit est de 10 000 Da à 30 000 Da et dans lequel un poids moléculaire moyen de l'alginate à poids moléculaire réduit est de 5 000 Da à 30 000 Da.

**2.** Pansement à base de collagène-alginate selon la revendication 1,
dans lequel le collagène à poids moléculaire réduit et l'alginate à poids moléculaire réduit sont obtenus en réduisant le poids moléculaire d'un collagène et d'un alginate en utilisant un processus de réduction du poids moléculaire à eau sous-critique, respectivement.

**3.** Pansement à base de collagène-alginate selon la revendication 1,
dans lequel un rapport en poids du collagène à poids moléculaire réduit et de l'alginate à poids moléculaire réduit est de 6 : 4 à 9 : 1.

**4.** Pansement à base de collagène-alginate selon la revendication 1,
dans lequel une capacité d'absorption du pansement à base de collagène-alginate pour l'exsudat est de 10 g/100 cm$^2$ ou plus, la capacité d'absorption étant mesurée selon le procédé de la description.

**5.** Pansement à base de collagène-alginate selon la revendication 1,
dans lequel une teneur en humidité du pansement à base de collagène-alginate est de 18 % ou moins, la teneur en humidité étant mesurée selon le procédé de la description.

**6.** Procédé de préparation d'un pansement à base de collagène-alginate, comprenant les étapes consistant à :

(a) mélanger un collagène à poids moléculaire réduit et un alginate à poids moléculaire réduit pour préparer un mélange réactionnel ; et
(b) ajouter un agent de réticulation au mélange réactionnel et réticuler le collagène à poids moléculaire réduit et l'alginate à poids moléculaire réduit pour obtenir le pansement à base de collagène-alginate selon l'une quelconque des revendications 1 à 5,
dans lequel un poids moléculaire moyen du collagène à poids moléculaire réduit est de 10 000 Da à 30 000 Da et dans lequel un poids moléculaire moyen de l'alginate à poids moléculaire réduit est de 5 000 Da à 30 000 Da.

**7.** Procédé selon la revendication 6,
dans (a), comprenant en outre d'obtenir le collagène à poids moléculaire réduit et l'alginate à poids moléculaire réduit par hydrolyse d'eau sous-critique d'un collagène et d'un alginate, respectivement.

**8.** Procédé selon la revendication 7,
dans lequel l'hydrolyse d'eau sous-critique est réalisée sous une pression de gaz inerte, de $N_2$ ou de $CO_2$.

**9.** Procédé selon la revendication 7,

dans lequel l'hydrolyse d'eau sous-critique est réalisée à une plage de température de 110 °C à 150 °C.

10. Procédé selon la revendication 6,
dans (b), comprenant en outre la réticulation du collagène r à poids moléculaire réduit et de l'alginate à poids moléculaire réduit et la lyophilisation.

11. Procédé selon la revendication 6,
dans lequel l'agent de réticulation est au moins un choisi parmi le formaldéhyde, le glutaraldéhyde, l'amidon de dialdéhyde, le 1-éthyl-3-(3-diméthyla-minopropyl) carbodiimide (EDC), le NHS, un composé époxy, le glyoxal de glutaraldéhyde, le glyoxal, la 2,2-diméthoxy-2-phénylacétophénone, le tripolyphosphate de sodium, le PEG de diacétaldéhyde, le scléraldéhyde, le squarate de diéthyle, l'épichlorhydrine la génipine, les tannins, les phényl-propanoïdes, les catéchines, le resvératrol, les flavonoïdes, et les isoflavonoïdes.

[FIG. 1A]

[FIG.1B]

[FIG.2A]

[FIG.2B]

[FIG.2C]

[FIG.2D]

[FIG.3]

A'          B'          C'          D'

[FIG.4A]

[FIG.4B]

[FIG.5A]

[FIG.5B]

[FIG.5C]

[FIG.5D]

**EP 4 088 755 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020160087033
- KR 1020170045458
- US 20170204136 A1
- US 20170182209 A1
- CN 108014366 A
- CN 107661229 A
- CN 109847097 A